# EUROPÄISCHE PATENTANMELDUNG

(11) **EP 2 281 534 A1**
(43) Veröffentlichungstag der Anmeldung: **09.02.2011**
(21) Anmeldenummer: 10405136.2
(22) Anmeldetag: 13.07.2010
(51) Int. Cl.: A61F 9/06

(54) **Schweisserschutzmaske**

(30) Priorität: 03.08.2009 CH 12082009
(71) Anmelder: OPTREL AG, 9630 Wattwil (CH)
(72) Erfinder: Brändle, Ignaz, 9607 Mosnang (CH)
(74) Vertreter: Frei Patent Attorneys

(57) **Zusammenfassung**

Eine Schweisserschutzmaske (1) weist einen tragenden Teil (2) mit einer Sichtöffnung (3) zur Aufnahme eines Filterelementes (5) als Blendschutz auf. Dabei ist an einer Aussenfläche des tragenden Teiles (2) ausserhalb des Bereiches der Sichtöffnung (3) mindestens ein Einsatzteil (6) angeordnet, welches in den tragenden Teil (2) einsetzbar ist und welches einen Teil der Aussenfläche des tragenden Teiles (2) bedeckt. Dadurch ist es möglich, Schweisserschutzmasken (1) mit geringem Aufwand zu individualisieren.

## Beschreibung

Die Erfindung bezieht sich auf eine Schweisserschutzmaske sowie auf einen Spannring gemäss dem Oberbegriff der entsprechenden unabhängigen Patentansprüche.

### STAND DER TECHNIK

Eine Schweisserschutzmaske oder ein Schweisshelm weist einen tragenden Teil mit einer Sichtöffnung auf, eine die Sichtöffnung abdeckende Schutzscheibe und ein hinter der Schutzscheibe angeordnetes, optisches Filterelement. Der tragende Teil von Schweissmasken oder Schweisshelmen dient neben seiner tragenden Funktion dem Schutz des Schweissers gegen mechanische Einwirkungen und gegen Wärmestrahlung. Dieser tragende Teil besteht üblicherweise aus einem Kunststoff, beispielsweise aus Polycarbonat und wird durch Spritzguss oder durch Pressen hergestellt. Das Filterelement ist in der Sichtöffnung des tragenden Teils angeordnet und dient dem optischen Schutz des Trägers, es stellt also den eigentlichen Blendschutz der Vorrichtung dar. Das Filterelement kann passiv sein, das heisst, es kann beispielsweise aus Schwarzglas bestehen. Das Filterelement kann auch aktiv sein, das heisst, es sperrt oder reduziert den Lichtdurchgang, wenn die Aussenlichtintensität eine vorgegebene Schwelle überschreitet. Die Schutzscheibe deckt die Sichtöffnung des tragenden Teils, derart, dass das Filterelement und gegebenenfalls der optische Sensor hinter der Schutzscheibe, das heisst, in getragenem Zustand zwischen der Schutzscheibe und den Augen des Schweissers positioniert sind. Die Schutzscheibe ist transparent und dient dem Schutz des optischen Filterelementes vor Verschmutzung und Beschädigung.

Es besteht das Bedürfnis, das tragende Teil individuell für eine Käuferfirma oder für einen einzelnen Benutzer zu gestalten. Dazu kann der tragende Teil bedruckt oder mit Aufklebern versehen sein. Die Materialien, welche für die Schutzfunktion der Maske erforderlich sind, lassen sich aber v.a. aufgrund von Form und Größe der Masken in der Regel schlecht bedrucken. Detailliertes Lackieren der Maske ist teuer, Aufkleber werden rasch unansehnlich und sind ebenfalls teuer.

### DARSTELLUNG DER ERFINDUNG

Es ist deshalb Aufgabe der Erfindung, eine Schweisserschutzmaske der eingangs genannten Art zu schaffen, welche die oben genannten Nachteile behebt.

Diese Aufgabe löst eine Schweisserschutzmaske mit den Merkmalen des Patentanspruches 1. Die Schweisserschutzmaske weist also an einer Aussenfläche des tragenden Teiles Einsatzteile auf, welche einen Teil der Aussenfläche des tragenden Teiles bedecken. Indem die Einsatzteile einsetzbar sind, ist eine einfache Individualisierung von Masken möglich. Eine begrenzter Satz von Masken (respektive tragender Teile) unterschiedlicher Farbe, kombiniert mit einer Vielzahl von unterschiedlich gefärbten oder bedruckten Einsatzteilen erlaubt eine entsprechende grosse Menge von Kombinationen. Sind die Einsatzteile nicht nur einsetzbar sondern auch auswechselbar, so kann die Individualisierung durch Auswechseln von Einsatzteilen leicht geändert werden.

Vorzugsweise beträgt die Grösse des mindestens einen Einsatzteiles 6 mindestens 10 cm² oder mindestens 15 cm² und bedeckt somit eine entsprechende Aussenfläche des tragenden Teiles. Eine maximale Fläche beträgt beispielsweise 300 cm².

Das Einsatzteil ist vorzugsweise in einer Vertiefung des tragenden Teiles angeordnet, wobei die Form der Vertiefung mit der Form des Einsatzteiles korrespondiert. Typischerweise ist die Form der Vertiefung kongruent zur Form des Einsatzteiles oder (im mathematischen Sinne) ähnlich zur Form respektive Kontur des flachen Einsatzteiles. Damit wird erschwert, dass irgendwelche Gegenstände zwischen den tragenden Teil und den Einsatzteil gelangen und den Einsatzteil ablösen. Die Vertiefung ist vorzugsweise etwa derart tief, dass das Einsatzteil mit seiner Aussenfläche zumindest annähernd mit der Aussenfläche des tragenden Teiles fluchtet. Die Fläche des tragenden Teiles, welche hinter das Einsatzteil zu liegen kommt, bleibt vorzugsweise - bis auf eventuelle Befestigungslöcher - geschlossen. Dadurch bleibt die Schutzfunktion des Helmes respektive der Maske erhalten, auch wenn keine Einsatzteile eingesetzt sind. Auch ist dadurch die Lichtdurchlässigkeit der Einsatzteile nicht von Belang, so dass diese aus einem hellen und damit einfach überdruckbaren Material, in der Regel ein Kunststoff, gefertigt sein können.

Das mindestens eine Einsatzteil weist vorzugsweise keine tragende Funktion auf. Es dient also weder einer signifikanten mechanische Stabilisierung des tragenden Teiles, noch als Halterung für weitere Elemente ausser für eventuelle Aufkleber oder Verzierungen. Typische Grössen für Einsatzteile sind minimale Breiten über 2 cm oder über 4 cm, und minimale Längen über 5 cm, sowie maximale Breiten bis 10 cm oder bis 15 cm sowie maximale Längen bis 10 cm oder bis 20 cm oder bis 30 cm.

In einer bevorzugten Ausführungsform der Erfindung sind die Einsatzteile aus einem anderen Material als der tragende Teil. Dadurch dass die Einsatzteile nicht aus dem Grundmaterial des tragenden Teiles gefertigt sein müssen, und das tragende Teil die optischen, mechanischen und thermischen Schutzfunktionen wahrnimmt, (Schlagfestigkeit, Resistenz gegenüber heissen Körpern, etc.) können die Einsatzteile aus kostengünstigen Materialien hergestellt werden.

In einer weiteren bevorzugten Ausführungsform der Erfindung weisen die Einsatzteile eine andere Farbe als der tragende Teil auf, und sind insbesondere intransparent. Dies kann realisiert werden, indem das Material der Einsatzteile durchgehend anders gefärbt ist, oder indem es eine andere Farbbeschichtung an der Oberfläche aufweist. In beiden Fallen ist dadurch eine einfache Individualisierung durch Wahl verschiedener Farben möglich. Bei der Produktion der tragenden Teile ist noch keine Individualisierung erforderlich, was die Produktion vereinfacht.

Beispielsweise kann der tragende Teil wegen seiner optischen Schutzfunktion dunkel eingefärbt sein, wohingegen die Einsatzteile aus einem hellen Kunststoff hergestellt sein können, der sich besser zum Bedrucken eignet.

Vorzugsweise sind die Einsatzteile relativ flach, und sind somit einfach zu bedrucken. Beispielsweise weicht die Aussenfläche des Einsatzteiles, über das ganze Einsatzteil gesehen, um weniger als 3 cm, insbesondere um weniger als 1 cm von einer Ebene ab.

In einer bevorzugten Ausführungsform der Erfindung ist das mindestens eine Einsatzteil irreversibel in den tragenden Teil eingesetzt. Es ist also nicht ohne Zerstörung des Einsatzteiles aus dem tragenden Teil entfernbar.

In einer anderen bevorzugten Ausführungsform der Erfindung ist das mindestens eine Einsatzteil lösbar im tragenden Teil eingesetzt. Die Einsatzteile sind vorzugsweise über Nocken, welche mittels Spannringen am tragenden Teil befestigt sind, gehalten. Dadurch ist eine mechanische Stabilität der Befestigung gegeben, so dass die Einsatzteile auch bei einem Sturz der Maske respektive des Helmes nicht wegfallen. Beispielsweise ist das Einsatzteil ausschliesslich mit Spannringen befestigt und liegen dazu vier bis fünf Spannringe pro Einsatzteil vor.

In einer anderen Ausfiihrungsform der Erfindung sind an einer Seite des Einsatzteiles eine oder mehrere Laschen ausgebildet, welche in korrespondierende Öffnungen oder Aussparungen des tragenden Teiles eingeführt sind und dadurch das Einsatzteil an dieser Seite halten. An einer gegenüberliegenden Seite des Einsatzteiles ist das Einsatzteil wie beschrieben mit einem oder mehreren Spannringen oder mit anderen Befestigungselementen am tragenden Teil befestigt. Die Spannringe sind vorzugsweise einstückig aus Kunststoff hergestellt.

Zur Befestigung mit einem Spannring weist ein Einsatzteil jeweils einen korrespondierend geformten Haltenocken auf, welcher durch eine Öffnung des tragenden Teiles geführt und von einem Spannring festgehalten ist. Der Spannring weist einen Andrückbereich zum Andrücken an eine im wesentlichen ebene Fläche und einen Sitzbereich zur Aufnahme einer Auskragung eines Haltenockens auf. Der Andrückbereich und der Sitzbereich sind, in Richtung einer Achse des Spannringes gesehen, gegeneinander versetzt angeordnet, und sind durch elastische Verformung des Spannringes gegeneinander verspannbar.

Der Spannring weist eine zentrale Halteöffnung auf, an welcher der Sitzbereich ausgebildet ist, und eine an die Halteöffnung anschliessende Einführungsöffnung, wobei der Innendurchmesser der Halteöffnung kleiner ist als jener der Einführungsöffnung. Dadurch ist ein pilzförmiger Haltenocken mit einer Auskragung durch die Einführungsöffnung führbar und mit der Auskragung seitlich in die Halteöffnung einschnappbar.

Die beschriebenen Spannringe sind grundsätzlich auch in anderen Einsatzbereichen, unabhängig von Schweisserschutzmasken, verwendbar. Sie lösen das Problem, eine Verspannung in Richtung der Längsachse einer Nocke zu erzeugen. In Verbindung mit einer Schweisserschutzmaske dient dies zum sicheren Festhalten eines Einsatzteiles an der dünnen Schale des tragenden Teiles, in anderen Anwendungen können andere Gegenstände mit entsprechenden Nocken an weiteren Objekten befestigt werden.

Weitere bevorzugte Ausführungsformen gehen aus den abhängigen Patentansprüchen hervor.

### KURZE BESCHREIBUNG DER ZEICHNUNGEN

Im folgenden wird der Erfindungsgegenstand anhand von bevorzugten Austührungsbeispielen, welche in den beiliegenden Zeichnungen dargestellt sind, näher erläutert. Es zeigen jeweils schematisch:
- Figur 1: drei Ansichten einer Schweisserschutzmaske;
- Figur 2: eine Detailansicht einer Innenseite einer Schweisserschutzmaske;
- Figur 3: eine Detailansicht mit Querschnitten durch eingeschnappte Haltenocken;
- Figur 4: einen Spannring mit eingeschnapptem Haltenocken; und
- Figur 5: verschiedene Ansichten und Schnitte eines Spannringes.

Die in den Zeichnungen verwendeten Bezugszeichen und deren Bedeutung sind in der Bezugszeichenliste zusammengefasst aufgelistet. Grundsätzlich sind in den Figuren gleiche Teile mit gleichen Bezugszeichen versehen.

### WEGE ZUR AUSFÜHRUNG DER ERFINDUNG

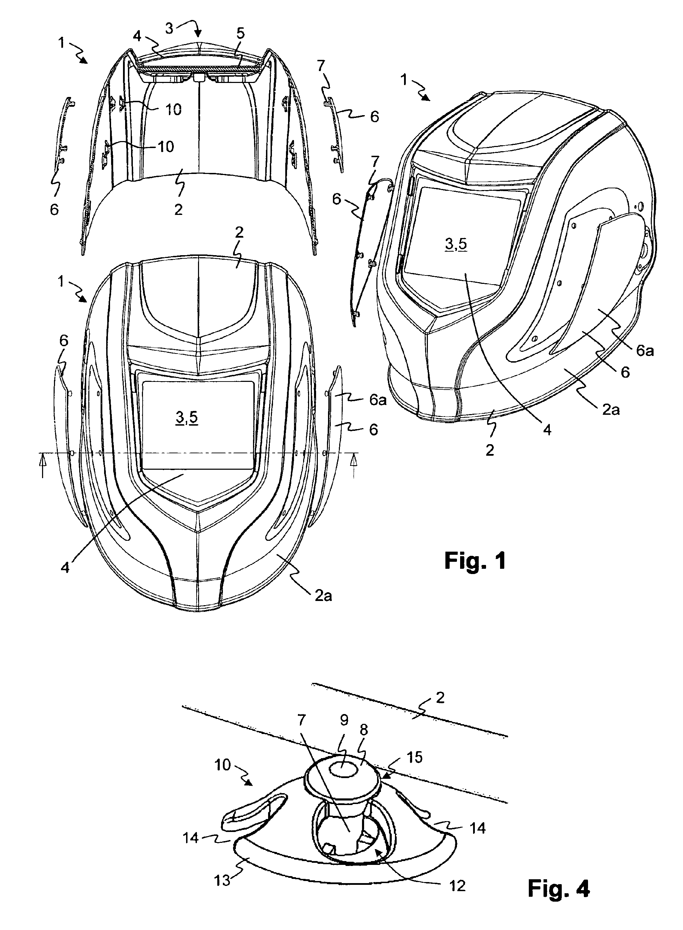

**Figur 1** zeigt drei Ansichten einer Schweisserschutzmaske 1, jeweils als Explosionszeichnung: eine Vorderansicht (links unten), einen horizontalen Schnitt (links oben) und eine perspektivische Ansicht (rechts). Eine Schweisserschutzmaske 1 weist einen tragenden Teil 2, auch Maskenkörper genannt, an welchem die übrigen Elemente befestigt sind, und welcher als Schutz vor Licht, Funkenflug etc. dient. Wenn die Schweisserschutzmaske 1 im oberen Bereich eher geschlossen ist, kann sie auch als Schweisserhelm bezeichnet werden. Am tragenden Teil 2, im Bereich einer Sichtöffnung 3, sind eine Schutzscheibe 4 und dahinter ein optisches Filterelement 5 als Blendschutz angeordnet. Zusätzlich sind zwei Einsatzteile 6 als Seitenteile links und rechts der Sichtöffnung 3, also im Wangen- und Schläfenbereich, angeordnet. In anderen Ausführungsformen der Erfindung liegen nur ein Einsatzteil vor, oder noch weitere Einsatzteile, beispielsweise im Stirnbereich über der Sichtöffnung 3, und/oder im Kinnbereich unter der Sichtöffnung 3.

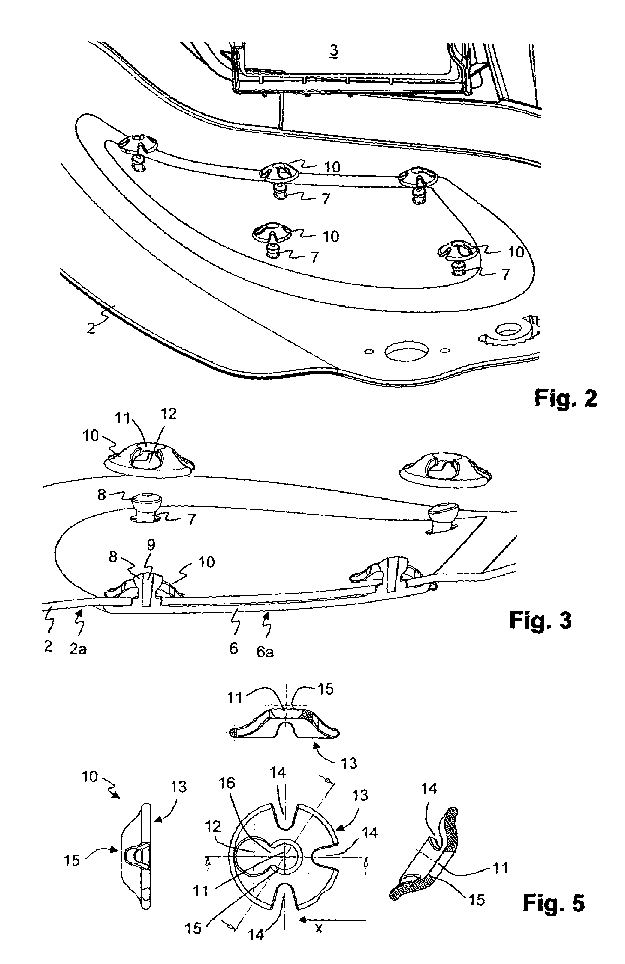

Die äussere Fläche des tragenden Teils 2 ist mit 2a bezeichnet, die äussere Fläche der Einsatzteile 6 mit 6a. Diese äusseren Flächen 2a, 6a fluchten vorzugsweise zumindest annähernd miteinander, indem die Einsatzteile 6 in korrespondierend geformten Vertiefungen des tragenden Teiles 2 angeordnet sind, wie auch in der Figur 3 gezeigt.

**Figur 2** zeigt eine Detailansicht einer Innenseite einer Schweisserschutzmaske 1, wobei Spannringe 10 wie bei einer Explosionszeichnung über zugeordneten Haltenocken 7 eines Einsatzteiles 6 gezeichnet sind. Die Haltenocken 7 sind durch korrespondierend angeordnete Löcher des tragenden Teiles 2 geführt und mittels der Spannringe 10 befestigt. Weitere Details betreffend die Befestigung und die dazu verwendeten Spannringe 10 sind anhand der folgenden Figuren erläutert: **Figur 3** zeigt eine Detailansicht eines tragenden Teils 2 mit einem eingesetzten Einsatzteil 6, mit Querschnitten durch eingeschnappte Haltenocken 7. **Figur 4** zeigt einen Spannring 10 mit eingeschnapptem Haltenocken 7. Der Haltenocken 7 weist eine Höhlung bzw. Ausnehmung 9 auf. **Figur 5** zeigt verschiedene Ansichten und Schnitte eines Spannringes 10.

Ein Spannring 10 weist einen Andrückbereich 13 zum Andrücken an eine im wesentlichen ebene oder leicht gekrümmte Fläche und einen Sitzbereich 15 zur Aufnahme einer beispielsweise pilzförmigen Auskragung 8 eines Haltenockens 7 auf. Der Andrückbereich 13 und der Sitzbereich 15 sind in Richtung einer Achse des Spannringes 10 gegeneinander versetzt angeordnet und sind durch elastische Verformung des Spannringes 10 gegeneinander verspannbar. Der Spannring 10 weist vorzugsweise eine rotationssymmetrische Grundform auf, mit einer zentralen Symmetrieachse. Der Andrückbereich 13 ist somit in Richtung der Symmetrieachse vom Sitzbereich 15 beabstandet. Der Andrückbereich 13 ist durch Einschnitte oder Ausnehmungen 14 unterbrochen, so dass der Sitzbereich 15, unter Aufdrücken dieser Ausnehmungen 14, gegen die Elastizität des Materials des Spannringes 10, gegen den Andrückbereich 13 gedrückt werden kann.

Der Sitzbereich 15 ist als Innenrand einer Halteöffnung 11 ausgebildet und korrespondiert in seiner Form mit der Form der Auskragung 8. Der innere Rand der Halteöffnung 11 verjüngt sich vorzugsweise von aussen nach innen, ebenso die äussere Kontur der Auskragung 8. Die Halteöffnung 11 weist an einer Seite, abseits der Symmetrieachse, eine Öffnung auf, welche über eine Verbindungsöffnung 16 in eine Einführungsöffnung 12 übergeht, deren Innendurchmesser grösser als der Innendurchmesser der Halteöffnung 11 ist.

Dadurch ist der Haltenocken 7 mit seiner Auskragung 8 durch die Einführungsöffnung 12 führbar (mit einer Bewegung in Richtung der Längsachse des Haltenockens 7). Anschliessend ist der Spannring 10 durch eine Bewegung normal zur Längsachse des Haltenockens 7 verschiebbar (in der **Figur 5** als Pfeilrichtung x angegeben). Dadurch schnappt der Haltenocken 7 in der Halteöffnung 11 ein, indem die Auskragung 8 auf dem Rand der Halteöffnung 11 aufliegt. Zum Einschnappen wird der Sitzbereich 15, unter Aufdrücken der Ausnehmungen 14, gegen den Andrückbereich 13 gedrückt, also der Spannring 10 etwas abgeflacht. Die Schnappverbindung zwischen Haltenocken 7 und Spannring 10 bildet sich also einerseits aus dem Zusammenwirken der Öffnung zwischen Halteöffnung 11 und Einführungsöffnung 12 mit den korrespondierenden Formen von Auskragung 8 und Sitzbereich 15, und mit der elastischen Nachgiebigkeit des Sitzbereiches 15 in axialer Richtung.

Beim eingesetzten Spannring 10 umschliesst die Halteöffnung 11 eine Auskragung 8 des Haltenockens 7 teilweise. Mit anderen Worten: Die Verbindungsöffnung 16 zwischen Einführungsöffnung 12 und Halteöffnung 11 ist etwas enger als die Dicke des Haltenockens 7 unterhalb der Auskragung 8, wie in den **Figuren 3** und **5** ersichtlich. Zum Einschnappen wird daher andererseits auch die Verbindungsöffnung 16 aufgeweitet, um das Durchschieben des Haltenockens 7 in Richtung senkrecht zur Längsachse zu erlauben.

Die Ausnehmungen 14 dienen vorzugsweise nicht nur zur Erhöhung der Nachgiebigkeit des Spannringes 10 in Richtung der Symmetrieachse, sondern auch zum Eingreifen eines Werkzeuges zum Einschieben und zum Abziehen der Spannringe 10 in Richtung senkrecht zur Längsachse der Haltenocken 7 respektive zur Symmetrieachse der Spannringe 10.

### BEZUGSZEICHENLISTE

| | | | |
|---|---|---|---|
| 1 | Schweisserschutzmaske | 8 | Auskragung |
| 2 | tragender Teil | 9 | Höhlung |
| 2a | Aussenfläche des tragenden | 10 | Spannring |
| | Teiles | 11 | Halteöffnung |
| 3 | Sichtöffnung | 12 | Einführungsöffnung |
| 4 | Schutzscheibe | 13 | Andrückbereich |
| 5 | Filterelement | 14 | Ausnehmung |
| 6 | Einsatzteil | 15 | Sitzbereich |
| 6a | Aussenfläche des Einsatzteiles | 16 | Verbindungsöffnung |
| 7 | Haltenocken | | |

## Patentansprüche

1. Schweisserschutzmaske (1), aufweisend einen tragenden Teil (2) mit einer Sichtöffnung (3) zur Aufnahme eines Filterelementes (5) als Blendschutz, **dadurch gekennzeichnet, dass**
an einer Aussenfläche des tragenden Teiles (2) ausserhalb des Bereiches der Sichtöffnung (3) mindestens ein Einsatzteil (6) angeordnet ist, welches in den tragenden Teil (2) einsetzbar ist und welches einen Teil der Aussenfläche des tragenden Teiles (2) bedeckt.

2. Schweisserschutzmaske (1) gemäss Anspruch 1, wobei das mindestens eine Einsatzteil (6) mindestens 10 cm² oder mindestens 15 cm² der Aussenfläche des tragenden Teiles (2) bedeckt.

3. Schweisserschutzmaske (1) gemäss Anspruch 1 oder 2, wobei das mindestens eine Einsatzteil (6) in einer Vertiefung des tragenden Teiles (2) angeordnet ist, wobei die Form der Vertiefung mit der Form des Einsatzteiles (6) korrespondiert.

4. Schweisserschutzmaske (1) gemäss einem der vorangehenden Ansprüche, wobei das mindestens eine Einsatzteil (6) keine tragende Funktion aufweist.

5. Schweisserschutzmaske (1) gemäss einem der vorangehenden Ansprüche, wobei das mindestens eine Einsatzteil (6) aus einem anderen Material als der tragende Teil (2) ist.

6. Schweisserschutzmaske (1) gemäss einem der vorangehenden Ansprüche, wobei das mindestens eine Einsatzteil (6) eine andere Farbe als der tragende Teil (2) aufweist.

7. Schweisserschutzmaske (1) gemäss einem der vorangehenden Ansprüche, wobei das mindestens eine Einsatzteil (6) intransparent ist.

8. Schweisserschutzmaske (1) gemäss einem der vorangehenden Ansprüche, wobei das mindestens eine Einsatzteil (6) im wesentlichen flach ist, insbesondere die Aussenfläche des Einsatzteiles (6), über das ganze Einsatzteil (6) gesehen, um weniger als 3 cm von einer Ebene abweicht.

9. Schweisserschutzmaske (1) gemäss einem der vorangehenden Ansprüche, wobei das mindestens eine Einsatzteil (6) irreversibel in den tragenden Teil (2) eingesetzt ist.

10. Schweisserschutzmaske (1) gemäss einem der Ansprüche 1 bis 8, wobei das mindestens eine Einsatzteil (6) lösbar im tragenden Teil (2) eingesetzt ist.

11. Schweisserschutzmaske (1) gemäss Anspruch 10, wobei das mindestens eine Einsatzteil (6) mit mindestens einem Spannring (10) befestigt ist, indem ein Haltenocken (7) des Einsatzteiles (6) durch eine Öffnung des tragenden Teiles (2) geführt und von einem Spannring (10) festgehalten ist.

12. Schweisserschutzmaske (1) gemäss Anspruch 11, wobei der Spannring (10) einen Andrückbereich (13) zum Andrücken an eine im wesentlichen ebene Fläche und einen Sitzbereich (15) zur Aufnahme einer Auskragung (8) eines Haltenockens (7) aufweist, und der Andrückbereich (13) und der Sitzbereich (15) in Richtung einer Achse des Spannringes (10) gegeneinander versetzt liegen und durch elastische Verformung des Spannringes (10) gegeneinander verspannbar sind.

13. Schweisserschutzmaske (1) gemäss Anspruch 11 oder 12, wobei der Spannring (10) aufweist: eine zentrale Halteöffnung (11), an welcher der Sitzbereich (15) ausgebildet ist, und eine an die Halteöffnung (11) anschliessende Einführungsöffnung (12), wobei der Innendurchmesser der Halteöffnung (11) kleiner ist als jener der Einführungsöffnung (12).

14. Einsatzteil (6) oder Satz von Einsatzteilen (6), welche zum Einsetzen in eine Schweisserschutzmaske (1) gemäss einem der vorangehenden Ansprüche geformt sind.

15. Spannring (10) zur Befestigung eines Einsatzteiles (6) an einer Schweisserschutzmaske (1) gemäss einem der Ansprüche 1 bis 13, aufweisend einen Andrückbereich (13) zum Andrücken an eine im wesentlichen ebene Fläche und einen Sitzbereich (15) zur Aufnahme einer Auskragung (8) eines Haltenockens (7), wobei der Andrückbereich (13) und der Sitzbereich (15) in Richtung einer Achse des Spannringes (10) gegeneinander versetzt liegen und durch elastische Verformung des Spannringes (10) gegeneinander verspannbar sind.
